# EUROPÄISCHE PATENTANMELDUNG

(11) **EP 1 300 465 A1**
(43) Veröffentlichungstag der Anmeldung: **09.04.2003**
(21) Anmeldenummer: 01123988.6
(22) Anmeldetag: 08.10.2001
(51) Int. Cl.: C12N 15/10

(54) **Verfahren und Vorrichtung zur Isolierung von RNS-Proben**

(71) Anmelder: Agrobiogen GmbH Biotechnologie, Larezhausen, 86567 Hilgertshausen (DE)
(72) Erfinder: Brem, Gottfried, Prof.Dr.Dr., 86567 Hilgertshausen (DE)
(74) Vertreter: Becker Kurig Straus

(57) **Zusammenfassung**

Die vorliegende Erfindung betrifft eine Vorrichtung zur Isolierung von RNS-Proben aus Tieren, wobei in dem Probensammelbehälter und in dem Probengewinnungsmittel Mittel zum Schutz vor RNS abbauenden Enzymen vorgesehen sind. Die vorliegende Erfindung betrifft insbesondere ein Verfahren zur Isolierung von RNS in biologischen Proben, wobei bereits in Neonaten eine Untersuchung auf RNS-Viren vorgenommen werden kann.

## Beschreibung

Die vorliegende Erfindung betrifft eine Vorrichtung zur Isolierung von RNS-Proben aus Tieren, umfassend einen Probensammelbehälter sowie ein Probengewinnungsmittel, wobei sowohl in dem Probensammelbehälter als auch in dem/am Probengewinnungsmittel Mittel zum Schutz vor RNS abbauenden Enzymen vorgesehen sind. Die vorliegende Erfindung betrifft insbesondere ein Verfahren zur Isolierung von RNS aus biologischen Proben, sowie zur Durchmusterung von Tierherden/-populationen auf Krankheitserreger.

Neugeborene Nutztiere werden gegenwärtig zur Sicherung bzw. zum Nachweis ihrer Identität und Herkunft mit von Hand beschrifteten oder vorbedruckten Kennzeichnungen versehen (Plastikbänder, Ohrmarken, Ringe etc.), um Verwechslungen mit anderen, im gleichen Umfeld geborenen Individuen zu vermeiden und die Neonaten ihren Müttern sicher zuordnen zu können. Ein Problem dieser extern angebrachten Kennzeichnungen besteht jedoch darin, daß sie verloren gehen, versehentlich oder sogar absichtlich ausgetauscht oder auf Grund mangelnder Dokumentation nicht mehr richtig zugeordnet werden können. In diesen Fällen ist eine zuverlässige Rekonstruktion der Identität des Neonaten und eine Zuordnung zu einer Mutter meist nicht mehr möglich.

Zur Lösung dieses Problem wird in der PCT/EP98/03075 eine Vorrichtungen vorgeschlagen, mit der gleichzeitig mit der Anbringung einer Ohrmarke eine DNS enthaltende, biologische Probe genommen wird, die in der Folge dazu verwendet werden kann, um beispielsweise den individualtypischen genetischen Fingerabdruck oder eine SNP-Signatur des Individuums zu bestimmen.

Im Rahmen des Geburtsgeschehens und der in nahezu allen Ländern vorgeschriebenen Markierung der Tiere können mit dieser Vorrichtung leicht Proben gewonnen und gelagert werden, die für Untersuchungen der genetischen Situation von Neonaten eingesetzt werden können.

Im Hinblick auf die gegenwärtig auftretenden seuchenartigen Erkrankungen bei Nutztieren besteht jedoch vermehrt ein Bedarf Tierherden, oder ganze Tierpopulationen, im allgemeinen auf Erkrankungen zu untersuchen. Ein im in diesem Bereich bislang ungelöstes Problem besteht jedoch darin, von Neonaten einer Herde bzw. auch Population Proben in ausreichender Qualität und vor allem Zuverlässigkeit hinsichtlich der Zuordnung zu den Individuen zu gewinnen, so dass auch Krankheitserreger, deren Nachweis aufgrund von Instabilität nur äusserst schwierig durchzuführen ist, erfasst werden können.

Ein umfassendes Neonaten-Screening würde es ermöglichen, eine Vielzahl von Erkrankungen rechtzeitig zu erkennen und damit auch konsequent therapieren oder bekämpfen bzw. Prophylaxemaßnahmen einleiten zu können.

Eine Aufgabe der vorliegenden Erfindung besteht daher darin, die bekannten Nachteile des Standes der Technik zu überwinden und eine Möglichkeit bereitzustellen, mit dem auch Krankheitserreger erfasst werden können, die aufgrund ihrer Instabilität nur schwer nachzuweisen sind.

Diese Aufgabe wird durch eine Vorrichtung gelöst, die einen Probenaufnahmebehälter und ein Probengewinnungsmittel enthalten, wobei sowohl in dem Probenaufnahmebehälter als auch an oder in dem Probengewinnungsmittel Mittel zum Schutz vor RNS-abbauenden Enzymen vorgesehen sind.

In den Studien, die letztendlich zu der vorliegenden Erfindung geführt haben, wurde nun gefunden, dass es offensichtlich nicht ausreicht, bei einer gewünschten Isolierung von RNA aus einem Individuum mit den herkömmlich verwendeten Vorrichtungen lediglich ein Mittel zum Schutz vor RNS-abbauenden Enzymen in dem zur Aufbewahrung der Probe bereitgestellten Behälter vorzusehen. Vielmehr muß sofort bei Kontakt des Probengewinnungsmittels mit der biologischen Probe sichergestellt werden, dass RNS-abbauende Enzyme keine Aktivität entfalten können, so dass die RNS-haltige Proben auch nach längerer Lagerung noch für Untersuchungen geeignet sind. Erfindungsgemäß wird daher auch das Probengewinnungsmittel mit einer derartigen Substanz versehen, so dass auch während der kurzen Zeit, während der das Probengewinnungsmittel beim Gewinnen der Probe in den Probenbehälter eingeführt wird, sichergestellt ist, dass kein wesentlicher Abbau von RNS stattfindet. Eine derartige Vorgehensweise stellt zudem sicher, dass die RNS auch bei längerer Lagerung in dem Behälter stabil bleibt und keinem Abbau unterworfen wird.

Der Probenaufnahmebehälter und das Probengewinnungsmittel können de facto jede geeignete Form annehmen, mit dem eine biologische Probe gewonnen und gelagert werden kann.

Gemäß einer bevorzugten Ausführungsform sind der Probenbehälter und das Probengewinnungsmittel wie in der PCT/EP/98/03075 beschrieben ausgestaltet, die hier zur weiteren Erläuterung der als Typi-Fix®Ohrmarken bezeichneten Vorrichtung zur Gewinnung von biologischen Proben unter Bezugnahme mitaufgenommen wird. Die Typi-Fix®Ohrmarken können beispielsweise mit jedem bekannten RNAse-Inhibitor, wie beispielsweise Trizol oder RNAlater™ (Fa. AMS Biotechnologie GmbH, Deutschland, Wiesbaden) befüllt oder beschichtet sein, die in den Probensammelbehälter und auf bzw. in das Probengewinnungsmittel eingebracht werden. Um sicherzustellen, dass daß sich die Flüssigkeit in dem Probensammelbehälter immer am Boden des Sammelbehälters befindet und dadurch mit der später gewonnenen Gewebeprobe zuverlässig in Kontakt kommt kann das Mittel vor RNS-abbauenden Enzymen beispielsweise durch eine in dem Behälter in Bodennähe vorgesehene Membran am Boden gehalten werden oder in dem Behälter kann ein Träger für das Mittel vorgesehen werden, beispielsweise ein poröser Träger, wie ein Schwämmchen, das ggf. in seiner Form der Form des Probensammelbehälters angepasst ist.

Um das Probengewinnungsmittel mit dem Mittel zum Schutz vor RNA-abbauenden Enzymen zu versehen, kann dieses beispielsweise mit dem entsprechenden Mittel beschichtet werden. Alternativ kann, wenn beispielsweise das Probengewinnungsmittel die Form einer Hohlspitze aufweist, die Spitze mit dem Mittel gefüllt und mit einem geeigneten Mittel, wie einer Membran verschlossen werden. Dies kann beispielsweise durch Ultraschallverschweißung einer abdeckenden Membran mit dem kegelförmigen Kopf des Dorns erfolgen oder auf andere geeignete Weise durch Zukleben etc. erreicht werden. Die derart präparierten Ohrmarken sind über Monate hinweg lagerbar, ohne daß das Konservierungsmittel seine Funktion einbüßt.

Der Vorteil der Beschickung sowohl der Hohlspitze des Dorns der männlichen Ohrmarke und des diese Spitze aufnehmenden Probensammelbehälters besteht darin, daß die Gewebeprobe dadurch beim Ausstanzen bereits von beiden Seiten mit der Konservierungsflüssigkeit in direkten Kontakt kommt und dadurch nicht nur von Flüssigkeit benetzt, sondern von der Flüssigkeit schnell duchdrungen und durchweicht wird. Durch dieses Vorgehen wird somit erreicht, daß die RNAsen umgehend inhibiert und die RNS in den Gewebeproben geschützt und in einem Ausmaß erhalten wird, so dass auch nach längerer Lagerung ein Nachweis bestimmter Transkripte in dem RNS-Pool noch möglich ist.

Gemäß einer Ausführungsform der Erfindung wird auch ein Verfahren zur Gewinnung von RNS-haltigen biologischen Proben bereitgestellt, bei dem die vorstehend beschriebene Vorrichtung zum Einsatz kommt. Das Verfahren eignet sich insbesondere zur Durchmusterung von Tierherden oder ganzen Tierpopulationen auf Krankhietserreger, wie RNS-Viren.

Mit Hilfe des hier entwickelten Verfahrens und den für die RNS-Gewinnung modifizierten Gewebeentnahme-Ohrmarken ist es dann beispielsweise möglich, Sanierungsprogramme für Viruserkrankungen durchzuführen, bei denen die Neonaten bereits das RNS-Virus enthalten. Durch Analyse der gewonnenen Gewebe/RNS-Proben können alle Virusträger identifiziert und aus der Herde entfernt werden. Dadurch würde die Infektionskette unterbrochen und der Bestand saniert werden.

Gemäß einer bevorzugten Ausführungsform liefert die vorliegende Erfindung ein Verfahren zum Nachweis von BVD bei Tieren. Dazu ist es notwendig, daß die das RNS-Virus enthaltende Probe innerhalb der ersten Lebenstage gewonnen wird, insbesondere bevor eventuell mit der Kolostralmilch aufgenommen Antikörper der Mutter wirken. Kolostrale Antikörper können den Virusnachweis etwa vom 3. Lebenstag bis zum Ende des 3. Lebensmonats derart beeinträchtigen, so daß ein negativer Befund in diesem Zeitraum nicht aussagekräftig ist und eine "diagnostische Lücke" besteht. Die vorliegende Erfindung ermöglicht daher erstmalig eine Untersuchung von Neonaten auf diese Erkrankung, so dass die bis dato bestehende "Lücke" geschlossen werden kann.

Gemäß Viehverkehrverordnung und EU-Richtlinie müssen alle in der EU geborenen Kälber innerhalb der ersten Lebenswoche mit zwei Ohrmarken gekennzeichnet werden. Wenn diese Kennzeichnung bereits zeitnah nach der Geburt durchgeführt wird und Ohrmarken verwendet werden, die die RNS in der Stanzprobe aus dem Ohr konservieren, kann von allen Kälbern einer Population Untersuchungsmaterial gewonnen werden, mit dem eine Virusdiagnostik möglich ist.

Alternativ und ergänzend können mit Probensammelbehältern aus dem Geburtsprobenentnahmeset auch Blutproben aus der Nabelschnur, Gewebeproben vom Nabelstrang und Plazentateilen, Schleimhauttupferproben etc. durch die bei der Geburt anwesenden Personen problemlos gewonnen werden. Der entscheidende Vorteil dabei ist, daß alle diese Probensammelbehälter und damit alle gewonnenen Proben durch die einheitliche und identische Kennzeichnung des Tieres mittels Ohrmarke und der Behälter mittels aufgedruckter Identifikation zweifels- und verwechslungsfrei gewonnen und im Labor mit minimalem Aufwand die Identität fehlerfrei (beispielsweise Identifikation mit Barcodeleser) weiterverarbeitet werden können.

Dadurch ist es möglich, auch alle persistent infizierten Tiere, die die Infektion in den Herden immer wieder anfachen könnten, zu entdecken. Entfernt man diese Tiere und ihre Mütter, kommt es zu keiner erneuten Re-Infektionen und nach 1 bis 3 Jahren sollten die einzelnen Herden und letztendlich dann auch die ganze Population von den entsprechenden Virusträgern befreit und saniert sein. Dies wäre volkswirtschaftlich für die Rinderzucht sehr wichtig. Nach Informationen der Bayerischen Landestierärztekammer ist BVD/MD in Bayern diejenige bovine Infektionskrankheit, die die größten wirtschaftlichen Verluste verursacht. Es gibt Modellrechnungen, die den Schaden, der durch BVD/MD entsteht mit 50 bis 100 DM pro Geburt kalkulieren. Daraus resultiert beispielsweise für die bayerische Rinderpopulation mit geschätzten 1,5 Millionen Geburten pro Jahr ein Schaden von etwa 100 Millionen DM. Neben den wirtschafltichen Aspekten wäre eine Sanierung unserer Rinderbestände von BVD/MD Infektionen auch im Sinne des Tierschutzes eine höchst anstrebenswerte Situation, weil damit den ansonsten erkrankten und an der Erkrankung verendeten Tieren viel Leid, Schmerzen und Schäden erspart werden können.

Auch andere Viren, die diaplazentar, intrauterin oder auf anderem Weg bereits vor, während oder unmittelbar nach der Geburt auf Neonaten übertragen werden können, können mit den in den Probesammelbehältern gewonnenen und konservierten Proben untersucht werden. Es gilt dies insbesondere auch für andere RNS-Viren, beispielsweise PRRS (Porcine Reproductive and Respiratory Syndrom), TGE (Transmissible Gastro Enteritis) etc..

Hinsichtlich der zu untersuchenden Individuen kommen alle Säugetiere in Betracht, für bestimmte Vorrichtungen und Untersuchungen auch Menschen, und aufgrund des wirtschaftlichen Interesses insbesondere Nutztiere, wie allgemein Ungulaten, beispielsweise Rinder, Schafe, Schweine, Pferde usw. im Vordergrund stehen.

Zur Durchführung des Verfahrens kann insbesondere ein Set von Probeentnahmevorrichtungen für verschiedenartige, im Rahmen des Geburtgeschehens leicht zu gewinnende Proben vorgesehen werden. Diese Proben können asserviert und außerdem auch in geeigneter Weise für eine spätere Bearbeitung oder Langzeitlagerung konserviert werden. Zur Vermeidung von Verwechslungen oder Vertauschungen sind die einzelnen Teile des Probenentnahmesets bis unmittelbar vor dem Gebrauch miteinander verbunden und verpackt. Sie werden erst bei der Benutzung (an ggf. vorhandenen Sollbruchstellen) voneinander getrennt. Alle Teile sind in unverwechselbarer Weise und dauerhaft vorgekennzeichnet (beispielsweise durch Laserbeschriftung) und alle Teile tragen sowohl diesselbe Analognummer zur visuellen Identifizierung als auch einen dazugehörigen Barcode, 2D-Code oder andere maschinenlesbare Kennzeichnungen (beispielsweise Find the Dot™).

Die Nummer des Probenentnahmesets wird der Identitätsnummer der Mutter zugeordnet und über die Probe von der Mutter auch genetisch mit ihr verknüpft.

Ein Probenentnahme-Set ist zusammengesetzt aus gekennzeichneten Probensammelbehälterund Kennzeichnungskomponenten.

Probensammelbehälter:
- Probensammelbehälter mit Konservierungsmittel für Gewebeproben zur Isolation von DNS
- Probensammelbehälter mit speziellem Konservierungsmittel (beispielsweise Trizol, RNAlater etc.) für Gewebeproben zur Isolation von RNS
- Probensammelbehälter mit Antibiotika- und Antimykotika-haltiger Zellkulturlösung zur Aufbewahrung von vitalen Zellen aus Gewebeproben von Tieren für die Zellanalyse (beispielsweise Viren) und -klonierung
- Probensammelbehälter für Blutstammzellen aus der Nabelschnur mit geeignetem Stabilisierungsmitteln zur Vorbereitung auf die Tiefgefrierkonservierung solcher Zellen als Stammzellquelle
- Probensammelbehälter für Blut, das aus der Nabelschnur ausgestreift wird
- Probensammelbehälter für Proben, die aus Teilen der maternalen oder fetalen Plazenta gewonnen werden
- Probensammelbehälter für Teile der Nabelschnur, die bei der Geburt abgetrennt werden
- Probensammelbehälter mit Konservierungsmittel für Blut, das durch Punktion von Blutgefäßen gewonnen wird
- Probensammelbehälter für Tupferproben, die aus Schleimhaut- oder Hautabstrichen oder Sekreten (Nase, Mund, Auge, Ohr, Genitalien etc.) gewonnen werden
- Probensammelbehälter für Kot (Mekonium) oder Urinproben, die vom Neonaten gewonnen werden
- Probensammelbehälter für Haare mit Haarwurzeln, die durch Auskämmen oder Ausreissen gewonnen werden
- Probensammelbehälter für geeignete, DNS enthaltende Referenzproben, die von der Mutter (Speichel-, Schleimhaut-, Blut-, Haar-, Gewebeprobe etc.) gewonnen werden
   und Alternativ, wenn möglich/gewünscht
- Probensammelbehälter für eine (DNS-haltige) Probe vom Vater (soweit dieser vorhanden oder greifbar ist) des Neonaten zur Abstammungsüberprüfung
- Probensammelbehälter für eine DNS-haltige Probe von den genetischen Eltern des Neonaten zur Abstammungsüberprüfüng in Embryo/Gametentransferprogrammen

Kennzeichnungskomponenten:
- Aufklebetiketten mit der Identitätsnummer, die abgezogen werden und zur Kennzeichnung von Transportverpackungen, Dokumenten, weiteren Sammelgefäßen etc. oder des Individuums verwendet werden können
- Dokumentationsblätter mit Identitätsnummer, in die alle relevanten Daten des Geburtsgeschehens eingetragen werden und damit eindeutig dem Neonaten zugeordnet werden
   und
- Plastikbänder, die die Kennzeichnung tragen und so gestaltet sind, daß sie nach einmaligem Schließen nicht mehr geöffnet werden können, ohne sie dabei zu zerstören; oder
- Metallmarken, Ringe o.ä. die in die Haut eingezogen werden können; oder
- Ohrmarken, die in ein oder beide Ohren eingezogen werden; oder
- andere Kennzeichnungssysteme wie beispielsweise elektronische Chips (RFID), Boli, Transponder oder sonstige Vorrichtungen, die mit der Identitätsnummer versehen werden können;
   oder
- Referenzkomponente mit Identifikationsnummer des Neonaten als Rückstellbeleg für die Mutter oder den Besitzer des Tieres

Für spezielle Anwendungen kann das Set entsprechend erweitert oder eingeschränkt werden. Jeder Anwender kann definieren, welche Probensammelbehälter und Kennzeichnungssysteme im Set enthalten sein sollen. Die einmalige Identifikationsnummer des Neonaten kann mit etwaigen vorhandenen Daten- oder Patientendokumentationssytemen elektronisch verknüpft bzw. dort eingespeichert werden.

Die Probensammelbehälter sind derart gestaltet, daß die Proben einfach und grundsätzlich auch von medizinisch nicht geschulten Personen gewonnen werden können. Eine Ausnahme stellt lediglich die - fakultative - Gewinnung von Blutproben aus der Punktion von Gefäßen dar. Diese wiederum ist nur dann unerläßlich, wenn Serumparameter oder sonstige Blutwerte bestimmt werden sollen. Dieser Vorgang obliegt aber ohnehin der (tier)-medizinischen Betreuung von Neonaten und wird vom Probenentnahmeset her insoweit unterstützt, als entsprechend vorgekennzeichnete Probensammelbehälter bereitgehalten werden.

Aus allen konservierten Proben kann bei der Aufarbeitung bzw. Untersuchung in Zweifelsfällen oder regelmäßig zusätzlich DNS isoliert und damit die Identität der Probenherkunft gesichert und überprüft werden. So kann sichergestellt werden, daß die einzelnen Proben bei der Untersuchung tatsächlich auch den richtigen Neonaten zugeordnet werden.

Weiterhin kann durch Vergleich der DNS von Neonatem und Mutter sichergestellt werden, daß der Neonat tatsächlich auch von der beprobten Mutter stammt und nicht verwechselt worden ist. Selbst bei Situationen, wie sie aus Embryotransfer resultieren, wo also die genetische Mutter nicht die gebärende Mutter des Neonaten ist, kann durch die DNS-Analyse aus planzentärem maternalen und fetalen Gewebe eine zweifelsfreie Verknüpfung hergestellt und fixiert werden.

Liegt eine DNS enthaltende Probe vom Vater vor oder kann sie gewonnen werden, ist eine zweifelsfreie Abstammungssicherung von beiden Eltern sehr einfach und kostengünstig möglich.

Die vorliegende Erfindung kann gleichermassen zur Gewinnung vitaler Zellen von einem Individuum genutzt werden. Dabei werden in dem Probenaufnahmebehälter und in dem Probengewinnungsmittel, vorzugsweise der Hohlspitze der Dornplatte, ein Zellkulturmedium bereitgestellt, vorzugsweise in der Art und Weise wie das Mittel zur Inhibierung von RNAsen, so dass bei Gewinnung der Probe das biologische Material sofort mit einem Medium in Kontakt tritt, in dem es seine Vitalität erhalten kann.

Das Medium enthält vorzugsweise Antibotika und/oder Antimykotika, so dass eine Kontaminierung der Kultur vermieden wird. Gleichermassen kann das Medium bereits Mittel enthalten, die ein Einfrieren der Zellen mit Erhalt ihrer Lebensfähigkeit ermöglichen, wie beispielsweise Glycerol oder DMSO.

Auf diese Art und Weise ist es nicht nur möglich Zellen vital zu erhalten und aufzubewahren und diese Zellen auch ggf. nach längerer Lagerung noch für weitere Experimente, wie Klonierungen zu verwenden. Vielmehr werden auch alle vorhandenen Antigene, sei es die von Krankheitserregern oder endogene Antigene in ihrer Struktur erhalten und können nach Wunsch mit den entsprechenden Mitteln nachgewiesen werden.

Die in den Probenaufnahmebehältern aufbewahrten Gewebeproben können jederzeit in Kultur genommen werden und den gewünschten Untersuchungen, beispielsweise einer Virusdiagnostik mittels FACS-Analyse unterzogen werden. Die Gewebeprobe kann zur besseren Bearbeitung mittels Kollagenasen oder anderen Enzymen behandelt, um die Gewebestruktur aufzulösen und die Zellen zu vereinzeln.

### Beispiel 1

Gewinnung und Untersuchung von RNS aus Hautstanzproben oder anderen mit dem Probensammelset gewonnenen RNS-haltigen Proben
Eradikation von BVD/MD (Bovine Virus Diarrhöe/Mucosal Disease)

Von Neonaten Kälbern wurden innerhalb der ersten Lebenstage mittels Trizol- und RNAlater-Typi-Fix®Ohrmarken Gewebe- und Sekretproben gewonnen. Die befüllten Probensammelbehälter mit der in das Konservierungsmittel eingebrachten Gewebeprobe wurden vor der Analyse bis zu 6 Wochen gelagert.

Zur RNS Extraktion wurden die Gewebeproben aus den Probensammelbehältern herausgeholt.

### 1. Bearbeitung mit klassischen Methoden

### RNS-Isolation

Zugabe von 100 µl Chloroform, >15 s Vortex, 2-3 min RT
15 min 10.000g, 4°C zentrifugieren
obere wässrige Phase abnehmen und in 0,25 ml Isopropanol aufnehmen
10 min RT, dann 10 min bei 10.000 g, 4°C abzentrifugieren,
Überstand abkippen, mit 0,5 ml 75% Ethanol waschen,
5 min bei 7500 g zentrifugieren,
Überstand abkippen und auf Eis offen trocknen

### Reverse Transkription (RT) und Polymerase-Kettenreaktion (PCR)

Die aus den in RNAlater™ Ohrstanzen mit peqGOLD Trifast isolierte BVDV-RNS wurde mittels reverser Transkriptase in komplementäre DNS (cDNS) umgeschrieben und im gleichen Ansatz in einer Polymerase-Kettenreaktion (one tube-RT-PCR) amplifiziert (Pfeffer et al. Vet Res Commun. 24 (7) (2000 Nov), 491-503; Kühne S., Diss. med. vet. München).

1 bis 5 µl RNS (1-3µg Gesamt-RNS) und je 20 bis 100 pmol forward- und reverse-Primer (primer 324 and 326, Vilczek et al. Arch.Virol.136:309-323) wurden in ein 0,2 ml Reaktionsgefäß überführt und mit Diethylpyrocarbonat- (DEPC-) behandeltem Wasser auf ein Volumen von 20 µl gebracht. Zu diesem Ansatz I wurden 80 µl Ansatz II pipettiert, der sich aus folgenden Komponenten zusammensetzte:
0,5 µl Reverse Transkriptase (RAV-2, 20 U/µl; Amersham Pharmacia Biotech)
0,5 µl Taq-Polymerase (AmpliTaq DNS Polymerase, 5 U/µl; Perkin-Elmer Biosystems)
0,5 µl Taq-Extender PCR Additiv (5 U/µl; Stratagene)
2,0 µl Nukleotidmix (dNTP-Mix: je 10 µmol/ml dATP, dCTP, dGTP, dTTP; MBI Fermentas)
0,5 µl Dithiothreitol (DTT, 100 mM; Roche)
0,5 µl RNAse-Inhibitor (RNSsin, 40 U/µl; Promega)
10,0 µl Reaktionspuffer (10x Taq-Extender-Puffer; Perkin-Elmer Biosystems)

Das Reaktionsvolumen wurde mit DEPC-behandeltem Wasser auf 80 µl aufgefüllt. Alle Arbeitsschritte fanden auf Eis statt. Die Reaktionsgefäße werden kurz geschüttelt und in einem Thermocycler Model 2400 (Perkin-Elmer Biosystems) nach folgendem Amplifikationsprogramm inkubiert:
- Reverse Transkription

Die Reverse Transkription erfolgte bei 50°C für 60 min., die Denaturierung und Trennung der Nukleinsäurestränge erforderte Temperaturen von 94°C für 30 s.. Bei 55 °C lagerten sich die Primer an die Sense- oder Antisense-Stränge an (Annealing); die DNS-Synthese (Elongation) fand bei 72 °C (10min.)
- PCR Zyklen
35 Zyklen, 30s bei 94°C, 2 min bei 55°C und 7 min bei 72°C
Termination: 20 min bei 72°C, Lagerung bei 4°C.

Nach der Amplifikation wurden 10 µl des Reaktionsgemisches mit 5 µl BSE-Puffer auf ein 1%-iges Agarosegel aufgetragen und bei 120 Volt in TAE-Puffer elektrophoretisch aufgetrennt. Das Gel wurde anschließend 15 min in einem Ethidiumbromidbad gefärbt und unter ultraviolettem Licht (UV) mit einer Wellenlänge von 1 = 301 nm ausgewertet. Die DNS-Banden wurden qualitativ mit dem Längenstandard (1kb DNS Ladder, New England BioLabs) verglichen und die Mengen abgeschätzt.

In gleicher Weise wie beschrieben wurde auch aus konservierten Schleimhaut- und Sekretproben und aus Nabelschnurgewebeproben (TFS-Gewbeentnahmesystem) RNS isoliert und das BVD Virus nachgewiesen werden.

### 2. Bearbeitung mit eigenen Verfahren

### Nachweis von BVDV in Typifix-Gewebeproben

Der Nachweis des BVDV in tierischem Gewebe wurde mit Hilfe einer RT-PCR geführt. Zunächst wurde aus den Proben die RNS gereinigt. Hierzu wurden Magnetpartikel mit einer Silica-Oberfläche verwendet. Ein spezifisches Fragment der viralen RNS wurde anschließend in zwei getrennten enzymatischen Reaktionen durch reverse Transkriptase in cDNA transkribiert und durch Polymerase-Kettenreaktion exponentiell vervielfältigt. Die Auswertung erfolgte mit Hilfe eines ELISA-artigen Verfahrens, bei dem die spezifischen Amplifikate direkt in den PCR-Gefäßen durch eine Biotin-Streptavidin-Erkennungsreaktion mit einem Enzymkonjugat und eine darauf folgende Enzymreaktion nachgewiesen wurden. Bei der Enzymreaktion wurde ein Substrat in ein farbiges Produkt umgesetzt. Die optische Dichte wurde dann in einem herkömmlichen ELISA-Reader gemessen.
- Reinigung von GesamtRNS aus Typifix-Proben

Die Gewebe-Proben wurden in RNS-later konserviert. Zum Aufschluß wurden die Proben in 1,5 ml Reaktionsgefäße mit Schraubverschluß überführt. In den Reaktionsgefäßen befanden sich Glaskugeln für den Aufschluß des Gewebes.

Zu den Proben wurden je 100 µl eines gebrauchsfertigen Lysepuffers A pipettiert. Die Gefäße wurden fest verschlossen und in die Halterung einer Schwingmühle (RETSCH) eingespannt. Der Aufschluß des Gewebes erfolgte anschließend mit Hilfe der Glaskugeln für 4 min. bei einer Frequenz von 30 sec-1 in der Schwingmühle. Die Reaktionsgefäße wurden anschließend kurz bei 5000 x g zentrifugiert. Danach wurden 900 µl Lysepuffer B zugegeben und die Lysate durch Invertieren der Reaktionsgefäße gemischt. Der Überstand über den Glaskugeln, die schnell absetzen, wurde mit einer Pipette abgenommen und in vorbereitete Reaktionsgefäße, die Magnetpartikel enthalten, überführt. Die Magnetpartikel wurden durch gründliches Vortexen im Lysismix resuspendiert. Um die RNS an die Magnetpartikel zu binden, wurden die Reaktionsgefäße anschließend 2 min. bei Raumtemperatur inkubiert. Zur Immobilisierung der Magnetpartikel wurden die Reaktionsgefäße danach in einen Magnetständer gestellt. Der Überstand wurde abgenommen und verworfen. Die Reaktionsgefäße wurden dann vom Magneten entfernt und in jedes Gefäß 500 µl eiskaltes, 70%iges Ethanol pipettiert. Die Magnetpartikel wurden durch Vortexen gewaschen und dann erneut im Magnetständer immobilisiert. Die ethanolhaltige Waschlösung wurde vollständig abgenommen und verworfen. Um Ethanolreste zu entfernen, wurden die Reaktionsgefäße aus dem Magnetständer genommen und geöffnet für 5 min. bei Raumtemperatur stehen gelassen. Danach wurden die Magnetpartikel in 60 µl RNAse-freiem Reinstwasser resuspendiert (durch kurzes Vortexen) und die RNS durch Inkubation für 2 min. bei 65°C in einem Thermoschüttler (Schüttelfrequenz 400 rpm) abgelöst. Die Reaktionsgefäße wurden anschließend für 2 min. auf Eis gekühlt. Die Magnetpartikel wurden nun erneut im Magnetständer immobilisiert, der RNS-haltige Überstand abgenommen und in saubere Reaktionsgefäße überführt. Während des Ansetzens der cDNA-Synthesereaktionen wurde die gereinigte RNS auf Eis gekühlt.
- cDNA-Synthese durch reverse Transkription

Die cDNA-Synthese erfolgte durch reverse Transkription mit MMLV Reverse Transkriptase (RNAse H minus). Als Primer wurde ein BVDV-spezifisches Oligonukleotid (BVDV 326) verwendet.

Zur Hybridisierung der Virus-RNS mit dem Primer wurden je 5 µl der gereinigten gesamtRNS-Präparate aus den Gewebeproben mit 2 µl Primer (10 pmol/µl) und 5 µl Reinstwasser in dünnwandigen 0,2 ml Reaktionsgefäßen gemischt. Die Gemische wurden anschließend in einem PCR-Cycler-Block für 10 min. bei 70°C inkubiert und danach schnell auf 10°C gekühlt. Während der Inkubation wurde ein Enzym-Puffer-Gemisch mit folgender Zusammensetzung vorbereitet:
4 µl RT-Puffer 5x konzentriert
1 µl dNTP-Gemisch (10 mM jedes der 4 Deoxynukleosidtriphosphate)
0,4 µl Enzymgemisch (bestehend aus 25 u/µl reverse Transkriptase und 10u/µl RNSsin)
2,6 µl Reinstwasser

Das Gemisch wird in Abhängigkeit von der Probenzahl mehrfach angesetzt.

Zu den gekühlten RNS-Primer-Gemischen (siehe oben) wurden 8 µl des vorbereiteten Gemisches pipettiert und kurz durch Vortexen vermischt. Anschließend erfolgte die cDNA-Synthese für 30 min. bei 37°C in einem PCR-Cycler-Block. Nach der Synthese wurde die reverse Transkriptase durch Hitze inaktiviert (5 min. bei 75°C). Im Anschluß wurde die cDNA unmittelbar auf Eis gekühlt und in einer PCR eingesetzt. Alternativ dazu können die cDNA-Proben bei -20 °C eingefroren und später weiter verarbeitet werden.
- Amplifikation eines spezifischen Fragments des BVDV-Genoms durch PCR und Auswertung

Von den cDNA-Präparaten wurde mit den genannten Primern ein Fragment des BVDV-Genoms in einer Polymerase-Kettenreaktion (PCR) vervielfältigt. In einer 96-well Platte, die aus 12 Streifen mit je 8 Reaktionshöhlungen zusammengesetzt war, wurde ein sogenannter PCR-Prämix pipettiert. Der Prämix bestand aus 2 Komponenten - dem PCR-Puffermix und dem PCR-Enzymmix. In den Gemischen waren Kit-artig alle Bestandteile für die PCR in einer an das Detektionsverfahren angepassten Form enthalten. An der Plastikoberfläche der Reaktionshöhlungen waren für den Nachweis des BVD-Virus spezifische Oligonukleotide kovalent gebunden. Sie wurden bei der Detektion als "Fänger" für BVDV-spezifische Amplifikate genutzt.

Entsprechend der Anzahl der zu analysierenden Proben einschließlich der zugehörigen Kontrollen wurde der Prämix mehrfach angesetzt. In einem 2 ml Reaktionsgefäß wurden je Probe 19,91 µl PCR-Puffer-Mix und 0,09 µl PCR-Enzymmix gemischt. Anschließend wurden je 20 µl des Prämix mit Hilfe einer Dispenser-Pipette auf die Reaktionshöhlungen der 8-er Streifen verteilt. Danach wurden je 2 µl der cDNA-Proben (siehe oben) in die Kavitäten pipettiert.

Die Platte wurde mit Nunc-Sealing-Tape dicht verschlossen und in den 96-er Block eines PCR-Cyclers (PE Biosystems, PCR System 9700) mit beheizbarem Deckel gestellt. Ein Programm mit folgenden Parametern wurde gestartet:
1. 94°C - 5 min.
2. 94°C - 30 sec.
3. 50°C - 30 sec.
4. 72°C - 30 sec. 35 Zyklen der Schritte 2-4
5. 72°C -2min.
6. 94°C - 3 min.
7. 56°C - 5 min.

Nach Beendigung des Programms wurden die Reaktionshöhlungen der Streifen in einem ELISA-Washer 5-fach mit Waschpuffer (Bestandteil des Kits) gewaschen. Reste des Waschpuffers wurden durch "Ausklopfen" auf Filterpapier entfernt. Mit Hilfe einer Multikanal-Pipette wurden anschließend in jede Kavität 50 µl eines gebrauchsfertigen Streptavidin-Peroxidase-Konjugats pipettiert und 15 min bei Raumtemperatur inkubiert. Danach wurde nicht gebundenes Konjugat durch 5-faches Waschen mit Waschpuffer entfernt. Nach erneutem "Ausklopfen" auf Filterpapier wurden in jede Kavität 25 µl einer Substratlösung (TMB) pipettiert und 15 min. im Dunkeln bei Raumtemperatur inkubiert. Das Substrat (TMB) wurde durch Peroxidase in ein blau gefärbtes Produkt umgesetzt. Die optische Dichte der blau gefärbten Lösung in den Kavitäten wurde in einem ELISA-Reader bei einer Wellenlänge von 650nm photometrisch gemessen.

Anhand der Messergebnisse für die Negativ-Kontrollen wurde statistisch ein Schwellenwert berechnet. Lagen die Messwerte für die Proben über dem Schwellenwert so war BVDV vorhanden gewesen. Durch entsprechende Positiv-Kontrollen wurde die Effizienz der RNS-Reinigung, cDNA-Synthese und PCR angezeigt.

### Auswertung

Bei allen Proben von persistent infizierten BVD Kälbern konnte das BVD Virus nachgewiesen werden. Ein Unterschied zwischen einer Konservierung mit Trizol und dem weniger toxischen und weniger leicht flüchtigen RNAlater war nicht festzustellen.

Es waren alle derzeit bekannten BVD Genotypen nachweisbar. Der Nachweis war nicht nur in Gewebeproben möglich, die innerhalb weniger Tage nach der Gewinnung untersucht wurden, sondern auch in den Proben möglich, die mit dem RNAlater TFS Probenentnahmesystem gewonnen und mehrere Wochen und Monate bei Raumtemperatur gelagert worden waren.

In allen Fällen war die Bande, die bei der Analyse der Gewebeprobe detektiert wurde, genauso aussagefähig wie die Banden der mitgeführten Positiv-Kontrollen aus Leukozyten-Pellet von Blutproben der Kälber. Die Sensitivität und Spezifität der BVD Diagnosen aus den Gewebeproben erlaubt eine Detektion von persistent infizierten BVD Kälbern und ermöglicht dadurch, diese Tiere frühzeitig und zuverlässig - vor der diagnostischen Lücke - zu identifizieren und durch Entfernung aus den Herden eine Sanierung dieser Herden - und bei populationsweitem Einsatz - eines ganzen Landes zu erreichen.

### Beispiel 2:

### Gewinnung von vitalen Zellen mittels Typi-Fix-Probensammelbehältern

Zur Gewinnung von vitalen Zellen ist es notwendig, die durch den Stanzvorgang erhaltene Gewebeprobe in einem geeigneten Medium aufzunehmen. Dazu wurden sowohl der Sammelbehälter als auch die Hohlspitze von Gewebeprobenentnahmesystemen mit Zellkulturmedium gefüllt und die Behälter dicht verschlossen. Als Medium wurde TL Hepes mit BSA, das Penicillin G (0,0325g/l) enthielt, dem Gentamicin (0,025 g/l) zugemischt wurde, verwendet.

Mit Medium gefüllte Sammelbehälter wurden verwendet, um von Rindern und Schafen Proben zu sammeln und ins Labor zu transportieren. Dort wurden die Proben aus den Sammelbehältern entnommen und nach bekannten Verfahren eine Zellkultur angelegt. Die in der Zellkultur wachsenden Fibroblasten wurden dazu verwendet, um über Nukleustransfer eine Klonierung durchzuführen. Nach Transfer der derart aus den konservierten Proben klonierten Zellen enstanden Graviditäten und Nachkommen, wobei das in der EP 98 907 950.4 offenbarte Verfahren, die hier unter Bezugnahme mit aufgenommen wird, eingesetzt wurde.

## Patentansprüche

1. Vorrichtung zur Gewinnung von biologischen Proben, umfassend einen Probensammelbehälter und ein Probengewinnungsmittel, wobei in dem Probensammelbehälter und dem Probengewinnungsmittel Mittel gegen RNS-abbauende Enzymen vorgesehen sind.

2. Vorrichtung nach Anspruch 1, wobei das Mittel vor RNS-abbauenden Enzymen in dem Probensammelbehälter in einer Einrichtung bereitgestellt ist, die am Boden des Probensammelbehälters angeordnet ist.

3. Vorrichtung nach Anspruch 2, wobei die Einrichtung eine Membran ist, durch die das Mittel zum Schutz vor RNS-abauenden Enzymen am Boden des Probensammelbehälters gehalten wird, oder ein poröser Träger.

4. Vorrichtung nach Anspruch 3, wobei der poröse Träger ein Schwamm ist.

5. Vorrichtung nach einem der vorhergehenden Ansprüche, wobei das Probengewinnungsmittel mit dem Mittel zum Schutz vor RNS-abbauenden Enzymen beschichtet ist oder einen Hohlraum aufweist, in dem das Mittel zum Schutz vor RNS-abbauenden Enzymen vorgesehen ist und der von der Umgebung durch eine Membran abgetrennt ist.

6. Vorrichtung nach einem der vorhergehenden Ansprüche, wobei das Mittel zum Schutz vor RNS-abbauenden Enzymen DEPC, Guanidiniumhydrochlorid, Trizol oder RNAlater ist.

7. Verfahren zum Nachweis eines Befalls eines Individuums mit RNS-Viren, wobei von dem zu untersuchenden Individuum mittels einer Vorrichtung nach einem der Ansprüche 1 bis 6 eine biologische Probe genommen wird, und die biologische Probe auf die Anwesenheit von RNS-Viren untersucht wird.

8. Verfahren nach Anspruch 7, wobei das RNS-Virus BVD, PRRS, TGE ist.

9. Verwendung einer Vorrichtung nach einem der Ansprüche 1 bis 6, zur Durchmusterung von Tieren auf RNS-Viren.

10. Verwendung nach Anspruch 9, wobei das RNS-Virus BVD, PRRS, TGE ist.
